# EUROPEAN PATENT APPLICATION

(11) **EP 1 693 460 A1**
(43) Date of publication of application: **23.08.2006**
(21) Application number: 04805071.0
(22) Date of filing: 26.11.2004
(51) Int. Cl.: C12N 15/67, C12N 1/21, A61K 48/00

(54) **METHOD OF REGULATING HETEROLOGOUS PROTEIN EXPRESSION CONTROLLED BY SALICYLIC DERIVATIVES IN MICRO-ORGANISMS ASSOCIATED WITH HIGHER ORGANISMS**

(30) Priority: 04.12.2003 ES 200302867
(71) Applicant: Universidad Pablo de Olavide, 41013 Sevilla (ES)
(72) Inventor: CEBOLLA RAMIREZ, Angel, E-41013 Sevilla (ES); ROYO SANCHEZ-PALENCIA, José Luis, E-41013 Sevilla (ES); SANTERO SANTURINO, Eduardo, E-41013 Sevilla (ES)
(74) Representative: Temino Ceniceros, Ignacio
(86) International application number: PCT/ES2004/000528
(87) International publication number: WO 2005/054477

(57) **Abstract**

The invention relates to a method which can be used to control the expression of heterologous proteins in micro-organisms using an expression system controlled by salicylate or derivatives. The genetic system can be used in attenuated pathogenic bacteria that can be induced once hosted by the eukaryotic cell by concentrations within the pharmacology safety range. The tropism of some bacteria for certain tissues or organs can be used to control the in-situ production of biomolecules for research purposes and as a system for the controlled release of biopharmaceuticals, e.g. in order to control the expression of anti-tumour proteins or antigens.

## Description

### FIELD OF INVENTION

The present invention relates to the field of genetic engineering. More specifically, this invention is related to the manipulation of gene expression in bacteria associated with cells of higher organisms using chemical compounds to induce heterologous gene expression.

### BACKGROUND OF THE TECHNIQUE

For biotechnologic applications in situ of live recombinant organisms, gene expression in microorganisms must be strongly regulated to avoid unnecessary and excessive metabolic wear (Glick BR. Biotechnol Adv. 1995,13:247-61). Environmental and intestinal flora can exercise strong selective pressure against strains that express heterologous genes constitutively. Even in pure cultures, mutants with a low expression level can come to dominate the culture within a few dozen generations. The expression of heterologous genes must be kept silent until activation is desired. The inducer signal should be specific, especially in complex environments, to avoid useless overexpression of the genes of interest. Two design alternatives are available for correctly controlling heterologous genes: i) Promoters regulated by environmental conditions, in which the environmental signal coincides with the site of action. ii) Chemically inducible promoters, where the compound is added at the time and place where activity is required. The second option is used preferentially. For example, the phenotype of an organism can be manipulated by adding a chemical compound, thus controlling either positively or negatively the appearance of the phenotype.

For biotechnologic applications, the ideal chemical inducer requires a series of characteristics: 1) the inducer should be inexpensive enough to be used in large amounts. 2) It should be compatible with the environment and/or biodegradable. 3) The inducer should be able to pass through diverse cell membranes if induction is to take place in endocytosed bacteria, or, generally speaking, bacteria hosted by higher organism.4) For bacteria hosted by higher organisms, potential pharmacologic effects on the system either should be minimized or should have relatively minor toxic effects.

The predominant regulatory systems capable of effectively controlling gene expression by chemical inducers are the *lacI*/*lac* systems in both prokaryotes and eukaryotes (Gossen M et al. Trends Biochem Sci. 1993, 18:471-5). These lactose operon-based systems have physiologic inducers that can be rapidly metabolized, such as lactose, and noncatalizable inducers, such as IPTG, which is suspected to produce toxic effects in mammalian cells.

Tetracycline, like other antibiotics, has been used as an effective chemical inducer in both prokaryotic and eukaryotic cells. This system consists of strong promoters that are repressed by the tetracycline-sensitive regulator TetR. One of the major problems is the use of antibiotic molecules for induction.

In Cebolla et al. (Appl Environ Microbiol. 2002, 68:5034-41 and J Biol Chem. 1997, 272: 3986-92), regulatory systems of xenobiotic compounds are described that can be activated by diverse salicylate derivatives, including one of the better known commercial drugs, acetylsalicylic acid (ASA). ASA is an inducer that meets all the requirements of an ideal chemical inducer for the chemical control *in situ* of heterologous gene expression.

The factors that control the naphthalene degradation pathway, NahR protein and its target promoters *Psal* and *Pnah,* have been used to express diverse heterologous genes in different bacterial strains, including *E. coli* (Cebolla A et al. Nucleic Acids Res. 2001, 29:759-66), *Bordetella* (Suarez A et al. Appl Environ Microbiol. 1997, 63:122-7), and *Pseudomonas* (Cebolla A et al. Nucleic Acids Res. 2001, 29:759-66). The *nahR*/*Psal* regulatory system has also been used as a biosensor that fuses to the *lux* operon of *Vibrio fischeri* (Burlage RS et al. J Bacteriol. 1990, 172:4749-57) and, similarly, as a recombinant protein production system (de Lorenzo V et al. Gene. 1993, 130:41-6). It has been demonstrated that the *nahR*/*Psal* system is finely regulated, exhibiting an induction ratio of 20 to 100-fold in response to its natural inducer, salicylate (Cebolla A et al. J Biol Chem. 1997, 272:3986-92). Vectors exist that establish the *nahR*/*Psal* regulatory system in the chromosome of gram-negative bacteria using minitransposons. It has also been used to express the LamB external membrane protein on the surface of *E. coli* and *Pseudomonas putida* (de Lorenzo V et al. Gene. 1993, 130:41-6; Cebolla A et al. Appl Environ Microbiol. 1996, 62:214-20). Other regulation systems inducible by aromatic compounds are also available, which can, in turn, be activated by acetyl salicylic acid. This is the case of XylS2, a mutant variation of the XylS regulator that recognizes and controls the expression of the meta operon in the catabolic pathway of toluene/xylene in *Pseudomonas putida.* This variant can recognize acetyl salicylic acid and induce expression 10-fold. The regulatory capacity of the *nahR*/*Psal* expression system can, in turn, increase 7 to 20 times (thus reaching 1,000-fold induction) using a regulation cascade system in which the *xylS2*/*Pm* regulators are placed downstream from *nahR*/*Psal* because both are activated simultaneously by salicylate and/or acetylsalicylic acid (patent US2001016354).

Other regulatory systems that respond to salicylate are systems derived from the MarR repressor and its target promoters (Martin RG and Rosner JL. Proc Natl Acad Sci USA. 1995, 92:5456-60; Sulavik MC et al. Mol Med. 1995, 1:436-46). These promoters are induced in the presence of salicylate, which inhibits binding to the operator. Purified MarR binds to salicylate with a Kd of 0.5 mM (Martin RG and Rosner JL. Proc Natl Acad Sci USA. 1995, 92:5456-60). The elements regulating response to salicylate have already been described in eukaryotic cells, especially plants in which salicylate is the main molecule in triggering signals that promote acquired systemic resistance. In plants, at least 5 factors that can interact with salicylate have been described, including kinases, salicylate binding proteins, etc. (Reymond P et al. Curr Opin Plant Biol. 1998, 1:404-11). Salicylate derivatives, particularly acetylsalicylic acid, apparently can be used as signal inducers using a large variety of available factors or artificial modifications of the same (Ramos JL et al. Proc Natl Acad Sci USA. 1986, 83:8467-71; Cebolla A et al. J Biol Chem. 1997, 272:3986-92).

Diverse regulatory systems have been used as expression systems for the production of recombinant proteins induced by salicylates or salicylate derivatives, such as the nahR/Psal regulatory system, other regulators of the lysR/nahR family, derivatives of the MarR repressor and target promoters, or the xylS2/Pm system or derivatives of the xylS/araC family of regulators.

The problem to be resolved by the present intervention is to provide a method for controlling the expression of heterologous genes in microorganism that are associated with cells of higher organisms, using a relatively well tolerated compound.

### SUMMARY OF THE INVENTION

The present invention describes a method for controlling the expression of heterologous genes in microorganisms associated with cells of higher organisms, including humans.

The present invention provides DNA sequences that encode regulators that respond to salicylate for the use of controlling the heterologous expression of genes in microbes that develop some type of association with a higher organism. The chemical compounds that activate the system can be transported actively or passively through biologic membranes and thus activate genetically manipulated strains, even when found inside a higher organism. The system of gene control described here is well-regulated by a chemical compound, in such a way that it reduces metabolic output because the action of the heterologous gene is null when no inducer has been added. The chemical compounds used in the invention as inducers are both salicylate and molecules derived from salicylate, such as acetylsalicylic acid or benzoate. These compounds have been tested for use in humans and have a low level of toxicity for therapeutic applications and well-defined pharmacokinetic and pharmacotoxicologic parameters.

The regulatory system can be introduced in a microorganism by means of plasmids or, preferentially, using integrative vectors to ensure the stability of the genotype (for example, Cebolla A et al. Appl Environ Microbiol. 2002, 68:5034-41).

In one embodiment of the present invention, this method consists of a regulatory system that can be controlled by a chemical inducer, a host microorganism containing the regulatory system that can interact or associate with cells of higher organisms, and one or more chemical compounds, which are salicylates or derivatives of salicylate, that act as an inducer. This system can be transported through the associated higher organism to activate or repress the expression of heterologous genes in the modified host microorganism.

In another, more preferred, embodiment of the present invention, the inductive chemical compound used is salicylate, anthranylate, 2-acetyl salicylate, 4-chloro-salicylate, 5-chloro-salicylate, 3,5-dichloro-salicylate, 5-methoxy-benzoate, benzoate, 3-methyl-benzoate, 2-methoxy-benzoate, 3-methyl-salicylate, 4-methyl-salicylate, 5-methyl-salicylate, or any other derivative of salicylate that maintains the C-1 carboxyl group in the aromatic ring or mixture of the same.

The bacterial cells maintain their viability and physical state because the expression of the heterologous genes is silenced. Administration of the drug enables their induction when one wishes to activate the expression of the heterologous genes presented, including in infected cells.

In another embodiment of the present invention, the regulatory system consists of at least one regulatory protein member of the LysR/NahR family of regulators.

In another embodiment of the present invention, the regulatory system consists of at least one regulatory protein that belongs to the AraC/Xy1S family of regulators.

In another embodiment of the present invention, the regulatory system consists of at least one regulatory protein that pertains to the MarR family of regulators.

In another embodiment of the present invention, the regulatory system consists of the *nahR*/*Psal* family of regulators or mutants of the same elements.

In another embodiment of the present invention, the regulatory system consists of the *xylS*/*Pm* family of regulatory systems or mutants of the same.

In another embodiment of the present invention, the regulatory system includes a circular genetic cascade that consists of an NahR transcriptional regulator, or mutant form of the same, and the XylS transcriptional regulator, or mutant form of the same, where the transcriptional regulators are placed in a hierarchical order in such a way that the transcriptional regulator NahR, or mutant form of the same, stimulates the expression of the transcriptional regulator XylS, or mutant form of the same, and the transcriptional regulator NahR, or mutant form of the same, and the transcriptional regulator Xy1S, or mutant form of the same, respond to the same inducer, which in this case is a salicylate or derivative, and a terminal target promoter that is sensitive in a dose-dependent way to transcriptional regulator XylS, or mutant of the same.

In a preferred embodiment of the realization of the present invention, the microorganism capable of interacting or associating with cells of higher organisms is a prokaryote.

In an even more preferred embodiment of the realization of the present invention, gram-negative bacterial cells are used.

In another, more preferred embodiment of realization, the genetic system is used in attenuated pathogenic bacteria, such as bacteria of the genus *Salmonella,* which can be induced when they become hosted by the eukaryotic cell by concentrations of the inducer chemical compound within the range of pharmacologic safety.

More specifically, the method of the present invention enables the control of the expression of therapeutic proteins or diagnostic proteins in attenuated bacteria associated with cells of higher organisms.

The expression of heterologous genes in microorganisms associated with cells of higher organisms allows the control of the production in situ of biomolecules for investigation as well as the control of the release of biodrugs, such as the control of the expression of antigens or antitumoral proteins, thanks to the tropism of some bacteria for certain tissues or organs, which can be used to increment the local concentration of recombinant proteins. Consequently, in a preferred embodiment of the present invention, a method of control of the expression of heterologous antigens is provided in an attenuated bacteria associated with cells of higher organisms for their use as a recombinant vaccine.

Under certain conditions, bacteria with tropism for tumoral cells (Pawalek et al. Cancer Res. 1997, 57:4537-44; Low et al. Nat Biotechnol. 1999, 17:37-41; Neumunaitis et al. Cancer Gene Ther. 2003, 10:737-44) can be used to program the release of proteins that inhibit the growth of tumoral cells, where those proteins can be produced under the control of a chemical inducer that can be administered at regular intervals to optimize the local concentration of the recombinant product.

In another preferred embodiment of the realization of the present invention, a method of control is provided for the expression of heterologous antitumoral proteins in attenuated bacteria associated with cells of higher organisms that have a certain affinity for tumoral cells in said higher organism.

In an even more preferred embodiment, the heterologous antitumoral protein consists of an interleukin, cytokine, toxin, cytotoxic protein, or antiangiogenic protein.

The capacity of salicylate and derivatives to pass through biologic membranes allows the system of expression of heterologous proteins to be induced by these compounds in the site of action, which in practice is the site of interaction between the bacteria and the cells of the host higher organism.

As understood in the present invention, association between a microorganism and a higher organism is any type of interaction between the microorganism and the higher organism, including symbiosis or pathogenesis. Consequently, the system of control of gene expression described in the present invention can be established in microorganisms, preferentially prokaryotic bacteria and, more specifically, in attenuated gram-negative bacteria, which can live inside or infect cells of eukaryotic organisms, including humans.

As understood in the present invention, salicylate derivatives are simple substitutions of the salicylate molecule that include at least a modification of the salicylate skeleton. They include, for example, compounds such as benzoate (substitution of―OH by ―H) or acetylsalicylic acid (-O-COCH₃ instead of―OH).

Vectors with regulatory systems that are strongly regulated by salicylate or its derivatives and can be integrated in the bacterial chromosome in a stable way have been developed previously (Cebolla et al. Appl Environ Microbiol. 2002, 68:5034-41).

The strongly regulated expression of toxic heterologous proteins by the host microorganism can be used to eliminate selectively the recombinant organisms used once these have completed their desired function, in order to modify the indigenous flora as little as possible. Therefore, in a preferred embodiment, the bacterial cells would contain a regulatory gene sensitive to salicylate or its derivatives and a target promoter, which controls the expression of a suicide gene that encodes a product toxic to the host cell, such as a restriction enzyme, toxin, antibacterial peptide, or other. This group of suicide genes include, for example, the kis/kid system (de la Cueva-Méndez et al. EMBO J. 2003, 22:246-251), colE3 (Diaz et al. Mol Microbiol. 1994, 13:855-861), etc.

The method of control of the expression of heterologous genes in microorganisms associated with cells of higher organism can be used to study the genes involved in pathogenesis by means of bacteria with conditional phenotypes. The action of the gene can be started or stopped at different stages of the biologic process. The needs of a particular gene for a certain process can be tested by controlling the presence of the inducer at a given stage of the process of association.

In another preferred embodiment of the present invention, the population of a given microorganism in bioreactors, such as fermenters, can be selectively eliminated by adding acetylsalicylic acid or an equivalent molecule. This system can also be used in gene therapy to selectively kill cells that may be selectively infected with a bacteria or virus that produces a toxic product or biomolecule capable of triggering apoptosis or any other mechanism of cell death in malignant cells.

In the present invention, the use of systems of expression inducible by salicylate derivatives to selectively kill recombinant organisms after the desired function has been carried out forms part of the protective measures of the invention. In the desired ambient, the selective death of microorganisms manipulated genetically would be triggered in the ambient to ensure that the indigenous flora be altered as little as possible. To achieve this, the cells would contain a regulatory system that responds to salicylate derivatives and a target promoter that controls that expression of a suicide gene that encodes for a toxic protein (for example, a restriction enzyme, toxin, antibacterial peptide, or other). The cells and bacteria and regulatory systems are either the *nahR*/*Psal* regulatory system or the *nahR*/*Psal-xylS2*/*Pm* cascade. A gene that encodes a toxin is available, for example, the bacteriokine colE3, a restriction enzyme such as EcoRI, downstream from the promoters *Psal, Pnah,* or *Pm.* The genetic constructs are introduced and inserted in the chromosome by recombination (Datsenko KA and Wanner BL. Proc Natl Acad Sci USA. 2000, 97:6640-5) or by transposition using a Tn5 minitransposon (Herrero M et al. J Bacteriol. 1990, 172:6557-67). Escindible antibiotic markers are used (Datsenko KA and Wanner BL. Proc Natl Acad Sci USA. 2000, 97:6640-5) to select the insertion in the chromosome of the suicide system that is controllable by salicylic derivatives, but later leaves the susceptible strain with the use of the same marker of kanamycin resistance, also making it more acceptable for its release into the environment. This way it can be used to control the death of live vaccines once they have been allowed to act for sufficient time.

In another embodiment of the present invention, a custom-designed bacteria is used that contains the selected regulatory system of the group, consisting of at least one regulatory protein that belongs to the LysR/NahR family of regulators, at least one regulatory protein that belongs to the AraC/XylS family of regulators, at least one regulatory protein that belongs to the family of MarR regulators, at least one *nahR*/*Psal* regulatory system, or mutants of the same elements, at least one *xylS*/*Pm* system of expression, or mutants of the same elements, a circular genetic cascade consisting of the NahR transcriptional regulator, or a mutant form, and the XylS transcriptional regulator, or a mutant form, or a combination of them, in the method of control of the expression of heterologous genes in the bacteria associated to cells of higher organisms. Preferentially, these heterologous genes encode for a therapeutic or diagnostic protein. In a preferred embodiment, this therapeutic protein is an antigen, antitumoral protein, or a mixture of the same.

Therefore, in a preferred embodiment of the present invention, the invention provides for the use of a system for regulating heterologous gene expression in bacteria associated with tumoral cells in a higher organism, in which said regulatory system is controlled by salicylate or its derivatives.

Likewise, the present invention also embodies the use of a system to regulate heterologous gene expression in bacteria associated with cells of higher organisms to control the expression of a heterologous antigen as a recombinant live vaccine.

### BRIEF DESCRIPTION OF THE DRAWINGS

Figure 1:
   A) Dose-response curve of SL7207 4S2 with different constructs of *Pm::trp'::'lacZ.* Inductions were performed in Luria Bertany medium at 30°C. After 4 hours in the presence of different concentrations of salicylate, enzyme activity was determined in Miller units (squares for pWSK-trp'::'lacZ and triangles for pCAS-trp'::'lacZ. The same results were obtained after 6 hours of induction.
   B) Detailed comparison of the ranges of induction obtained after inducing SL7207 4S2 with salicylate 2 mM for 4 hours (n=3, error bars show the standard deviation). Although the total production of β-galactosidase obtained with pCAS-trp'::'lacZ was greater, due to the low baseline levels of pWSK-trp'::'lacZ the induction range was 10 times greater.
Figure 2:
   SL7207 4S2 infecting HeLa cells that express β-galactosidase after induction with salicylate or acetyl salicylic acid (2 mM). In A) high baseline levels of SL7207 4S2 pCAS-trp'::'lacZ are evident, while in D) the baseline levels of pWSK-trp'::'lacZ are undetectable. However, large differences are appreciated in the levels induced by the two vectors―high and low number of copies―B, E). In addition, ASA and salicylate induce similar levels of β-galactosidase C, F).
Figure 3:
   FACS analysis of F1.A11 cells infected with SL7207 4S2 pWSK (A) or pWSK-GFP (B and C). After infection, cell cultures were induced with salicylate 2 mM in culture medium for 4 hours (A and C). The diagrams show the number of cells selected plotted against the fluorescence obtained by GFP (FL1).
Figure 4:
   FACS analysis of a spleen cell suspension obtained from a mouse infected with SL7207 4S2 pWSK (A)and induced with 2 mg of salicylate administered intraperitoneally, not induced with pWSK-GFP (B) and induced with pWSK-GFP (C). Diagrams show the number of cells selected plotted against fluorescence obtained with GFP (FL1).
Figure 5:
   Flow cytometry analysis of a cell suspension obtained from mesenteric nodes of mice previously infected with SL7207 4S2 pWSK (A), and induced intraperitoneally with 2 mg of salicylate, pWSK-GFP but not induced (B) or induced (C).
Figure 6:
   Uptake of tritiated thymidine by CD4+ T cells obtained from the spleen of mice infected orally with 5x10E8 SL7207 4S2 pWSK and induced (group 3), with pWSK-trp'::'lacZ and not induced (group 2), or with pWSK-trp'::'lacZ and induced (group 1).
Figure 7:
   Flow cytometry analysis of cell suspensions of fibrosarcomas produced subcutaneously. The mice that developed tumors were infected with SL2707 4S2 with pWSK-GFP and not induced (gray curve) or induced intraperitoneally with 2 mg of salicylate (white curve).

### EXAMPLES:

EXAMPLE 1. Control by salicylate and acetyl salicylic acid of the overproduction of heterologous genes in *Salmonella.*

In this example, the attenuated strain of *Salmonella typhimurium* SL7207 was used, which has been used previously as a vaccine carrier in different experiments (Darji A et al. Cell. 1997, 91:765-75; Paglia P et al. Blood. 1998, 92:3172-6; and Medina E et al. Eur J Immunol. 1999, 29:693-9). The strain was manipulated genetically to insert the regulatory modulator of the amplification circuit in cascade described above in the present report. This example illustrates a method by which heterologous overexpression of genes was regulated with acetyl salicylic acid and salicylate as inducers of bacteria capable of infecting mammalian cells.

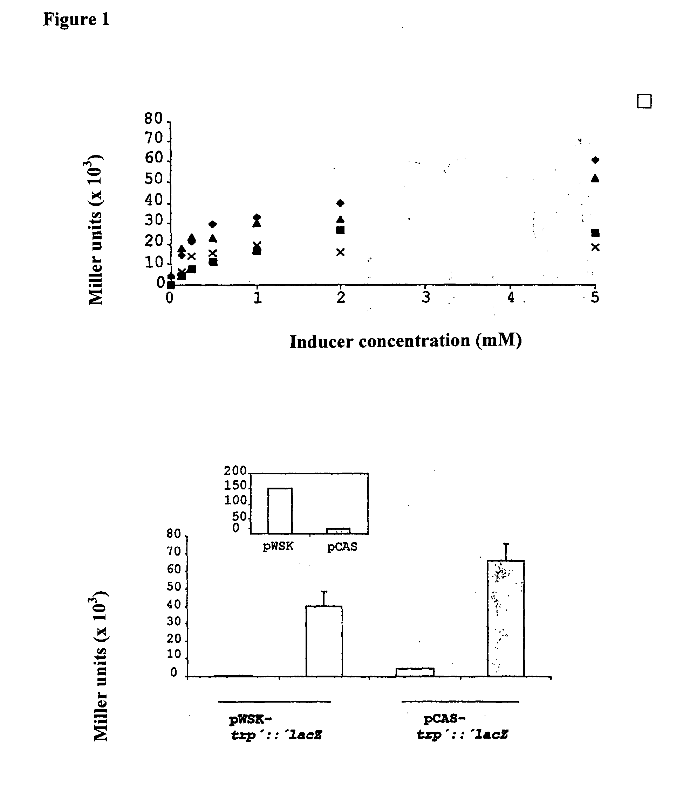

To test the amplification potential on gene expression provided by the cascade circuit dependent on salicylic derivatives, the regulator *nahR*/*Psal::xylS2* was inserted in the chromosome of SL7207 as described below. A spontaneous SL7207 mutant resistant to rifampicin was used as a conjugation receptor with S17-1λ*pir* pCNB4S2. Conjugation was performed in LB medium, 1% sodium citrate, for 3 hours at 30°C. The transposition events were isolated by overnight incubation in LB medium with rifampicin 20 µg/mL and kanamycin 50 µg/mL. Ampicillin-susceptible colonies (100 µg/mL) were obtained. The resulting strain, SL7207 4S2 was electroporated with either pCAS-trp'::'lacZ or pWSK-trp'::'lacZ. These plasmids contain the trp'::'lacZ fusion promoter Pm in high-copy and low-copy vectors, respectively. Cultures were grown at 37°C and 150 rpm in LB ampicillin until reaching a DO660 of approximately 0.3. The necessary amount of inducer was added and cultures were incubated again at 37°C until Miller unit measurements were made. As observed in figure 1, both salicylate and acetylsalicylic acid induced the system similarly, independently of the plasmid used. The results obtained at two time points (4 and 6 hours reinduction) were identical, showing that the system was fully induced in 4 hours.

Upon reducing the number of copies of marker plasmid (Fig. 1b), 10-fold lower baseline levels were obtained (4384 + 388 vs. 204 + 6 Miller units, n=3), while maintaining most of the induction capacity. Once induced (2 mM salicylate), the effect of synergic amplification of the expression system allowed the vector based on pWSK to attain enzyme activities similar to those obtained with vector pCAS (36,792 + 798 vs. 66,185 + 8171, respectively). The induction range of pWSK was 10 times greater than the induction range of pCAS (180 vs. 15). These results made it preferable to use SL7207 pWSK-trp'::'lacZ for the following experiments. As growth inhibition was evident at high concentrations (5 mM) of acetylsalicylic acid, 2 mM was used for the detailed experiments in vitro with cell cultures.

Therapeutic doses of salicylate measured in plasma are higher than 4.6 mM. Given that similar levels of expression are attained with 2 mM and 5 mM, we decided to use the higher dose for experiments in vivo because the reported half-life is 2-4 hours. Thus, a single dose of 5 mM yields at least 4 hours of activation.

EXAMPLE 2. Use of circuits of response to salicylate derivatives to induce gene expression in infected cells.

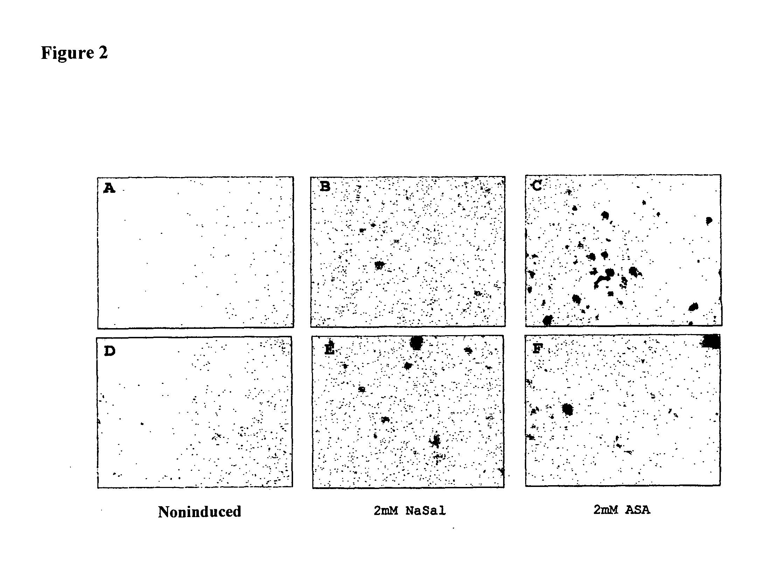

This example shows how the expression of β-galactosidase can be controlled in bacterial cells that infect mammalian cells by adding salicylate derivatives. SL4S2 bearing pWSK-trp'::'lacZ and pCAS-trp'::'lacZ was used to infect mammalian cells (Royo JL et al. submitted), as is described again below. Both HeLa cells and J774.A1 macrophages were cultured on 24-well plates with DMEM medium, L-glutamine, and 10% fetal goat serum. Cell cultures were incubated at 37°C with 6% CO₂ until a confluence of almost 80% was reached. *Salmonella* cultures were grown overnight at 37°C, 15m rpm in LB 0.3 sodium chloride medium with ampicillin 100 µg/mL as needed. Each well was infected with 10E6 bacteria from a saturated culture and incubated at 37°C, 6% CO₂, for 1 hour (J774A.1) or 2 hours (HeLa). Cells were washed 3 times with PBS before adding new DMEM supplemented with gentamicin 50 µg/mL to kill the bacteria remaining outside and with ampicillin to avoid plasmid loss. When required, either salicylate or acetyl salicylic acid was added to reach a final dose of 2 mM. After 4 hours of incubation (37°C, 6% CO₂), the cell cultures were washed with PBS, fixed with 2% paraformaldehyde during 20 minutes, and stained with a β-gal staining kit (Roche) following the manufacturer's instructions. The expression of β-galactosidase confirmed that both salicylate and acetyl salicylic acid enter the bacterial cytoplasm. Once inside the cell, both inducers activate the signal cascade to similar levels (Figure 2b, 2c, 2d, and 2f). Baseline levels of pCAS-trp'::'lacZ were detected easily. However, baseline lacZ activity produced by the low-copy number vector was undetectable (Figure 2a and 2d). These experiments showed that both molecules can diffuse and enter the bacterial cytoplasm, thus activating the expression system. In addition, the eukaryotic ambient does not interfere significantly with gene overexpression, probably because the components of the cascade system are active over a wide range of metabolic conditions. Although the same results were observed with J774A.1 macrophages, some cell death caused by the infection itself was observed.

EXAMPLE 3. Use of circuits of response to salicylic derivatives to control expression in tumoral cells.

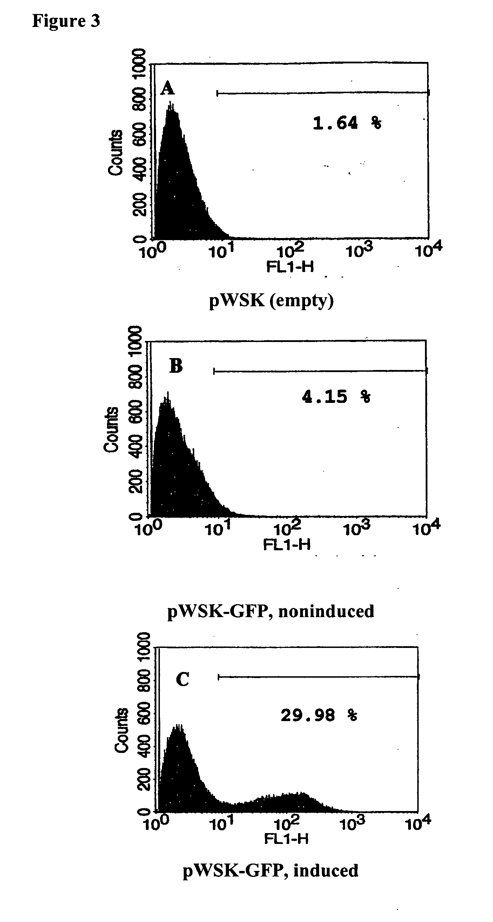

In this example, the capacity to control gene expression using salicylate and the system of expression in cascade is confirmed using green fluorescent protein as a marker gene. To avoid potential metabolic barriers, SL7207 4S2 with pWSK-GFP was used to monitor GFO expression in vitro. An experiment could also be performed to characterize SL7207 4S2 gene expression in the tumoral line used as a model. F1.A11 is a highly tumorigenic and metastatic cell line derived from murine fibrosarcoma of the Balb/c strain. Metastases are easily located because F1.A11 expresses and presents β-galactosidase as a tumor marker (Paglia P et al. Blood. 1998, 92:3172-6 and internal references). They were infected with almost confluent cultures of F1.A11 with SL7207 4S2 with either pWSK-GFP or its corresponding empty vector. Induction was done as described below. After 4 hours of induction with salicylate 2 mM, cells were collected, washed, and analyzed by flow cytometry. Figure 3 shows the cell event count plotted against the relative fluorescence (FL1) of cells infected under different conditions. Fluorescence varied between 11.72 (empty vector) and 12.51 (infected with SL7207 pWSK-GFP). When cultures were examined under the microscope, no GFP was observed in the empty vector or noninduced pWSK-GFP. After adding salicylate 2 mM (Figure 3c), fluorescence rose to 116.7 (a 10-fold increase). After examining the data as a whole, SL7207 4S2 was judged to be a good candidate for more in-depth studies.

EXAMPLE 4. Control in vivo of gene expression in infected animals.

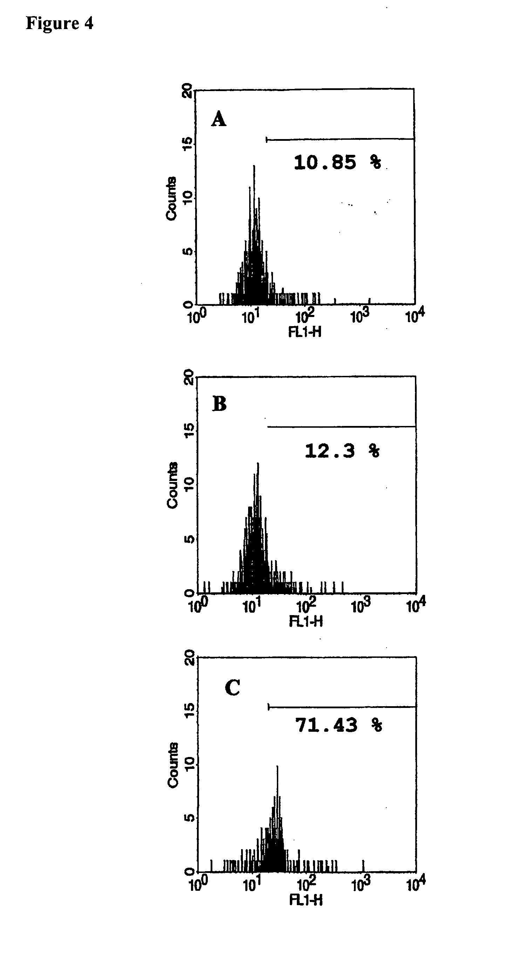

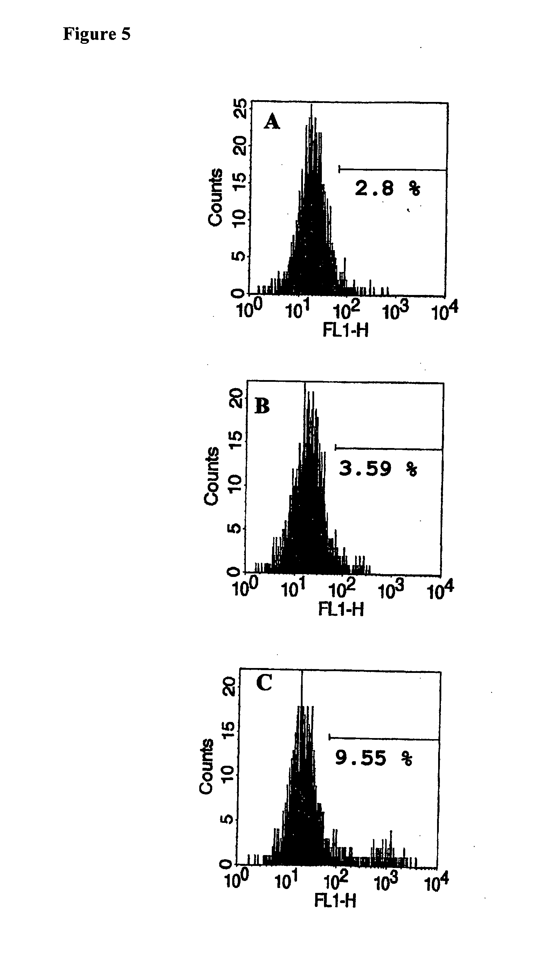

In this example, salicylate response circuits were used to control the gene expression of microbes infecting live animals. Colony-forming units (10E6) of attenuated *Salmonella* bearing the cascade circuit module (SL7207 4S2 with pWSK-GFP) were administered intraperitoneally (i.p.) (in 150 µl of PBS). Thirty minutes after infection, 2 mg of salicylate was injected intravenously (i.v.) or intraperitoneally to reach 5 mM salicylate. After 4 hours of incubation, animals were sacrificed and peritoneal exudates, spleens, and mesenteric lymph nodes were analyzed. Highly GFP-positive cells were found in mesenteric lymph nodes (Figure 5) and spleen (Fig 4), compared to cells not induced or induced with the empty vector. Cells positive for GFP were not seen in peritoneal exudates, revealing either migration or apoptosis of peritoneal macrophages. Identical results were obtained with both i.p. and i.v. induction.

EXAMPLE 5. Control in vivo of antigen expression in infected animals.

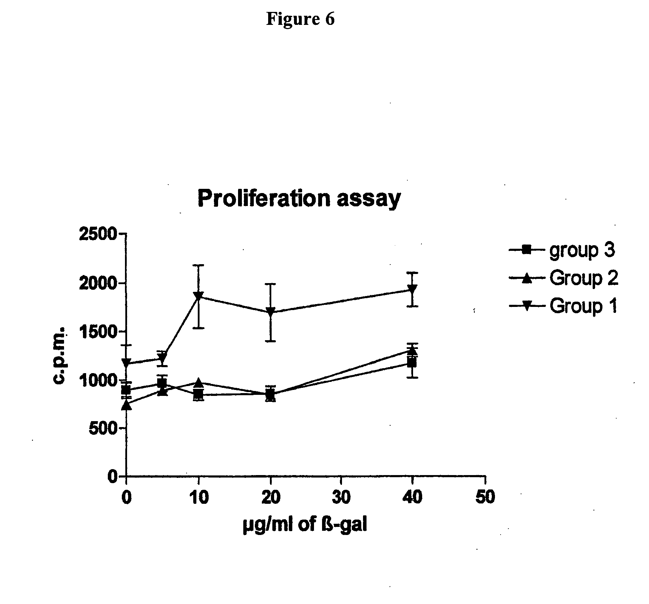

In this example, an attenuated bacterial strain was used as a vector to present heterologous antigens to the immune system of the mouse in a way conditional to acetylsalicylic acid administration. A protective immune response can be elicited using different attenuated strains derived from pathogenic wild strains such as enteric *Salmonella* serovar *typha* and *typhimurium* (*S. typhi* and S. *typhimurium*). In these experiments, mice were immunized with 5x10E8 SL7207 4S2 bearing the plasmid pWSK or pWSK-trp'::'lacZ. Twelve hours after immunization, 2 mg of salicylate was administered i.p. Equal doses of salicylate were given on days 7 and 14 after the challenge. Two weeks after the last induction, animals were sacrificed. Proliferation assays were performed with spleen cells. In figure 6, it is evident that restimulation in vitro of CD4+ T cells after the addition of pure β-galactosidase differed significantly between induced and noninduced mice. This experiment confirmed that SL7207 4S2 activated the *nahR*/*Psal* and *xylS2*/*Pm* modules, thus promoting the expression of β-galactosidase. This signal can therefore conditionally modulate immune response in mice.

EXAMPLE 6. Regulated intratumoral expression of recombinant proteins.

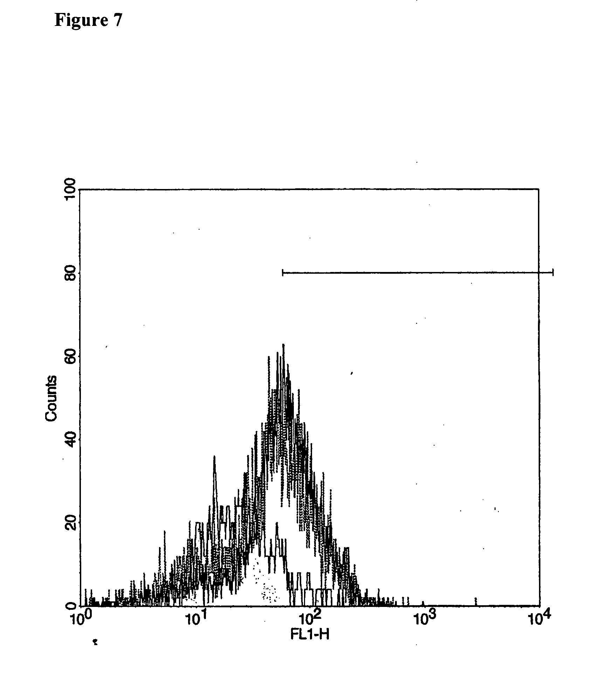

In this example, we measured the tropism of certain microbes for tumors or other diseased tissues to control the release of protein expression. This example shows how protein expression can be concentrated in cells where therapeutic proteins can be produced after the administration of acetyl salicylic acid. Tumors are generated by subcutaneous injection of 10E4 F1.A11 cells in the left lower flank of 9-week-old female Balb/c mice. When the tumors were palpable (100-200 mg), 10E6 colony-forming units of SL7207 4S2 with pWSK-GFP and the corresponding empty vector were administered intraparenterally. On day +4, the mice were divided into different groups. Some were treated with a dose of 5 mM salicylate i.v./i.p. and others were kept as noninduced controls. Animals were sacrificed four hours after induction and the spleen, mesenteric lymph nodes and tumors were separated aseptically for analysis. The cell suspensions of independent tumors that had been treated with salicylate showed a positive phenotype on FL1 (Fig 7). Cells in the FL1 > 10E2 window rose from 11% to 39-48%, showing a behavior that paralleled infections in vitro. Other tumors were fixed and stained to examine the presence of SL7207 4S2. Organs were weighed, homogenized, and resuspended in sterile PBS before seeding. In some assays, ratios varied from 4:1 to 850:1, in accordance with what has been described previously as positive tropism (Pawelek JM et al. Cancer Res. 1997 Oct, 57:4537-44; Low KB et al. Nat Biotechnol. 1999, 17:37-41; and Neumanaitis J et al. Cancer Gene Ther. 2003, 10:737-44). On the other hand, without taking into account high GFP expression localized in the tumor, no tropism was observed in various mice. This observation has been described previously and could be due to differences in the nature of the tumorigenic lines, the genetic background of the mice, the *Salmonella* strain used, or simply stochastic variations attributed to the phenomenon of tumoral progression.

## Claims

1. A method for controlling the expression of heterologous genes in microorganisms associated with cells of higher organisms consisting of:
a. a regulatory system that can be controlled by a chemical effector;
b. a host microorganism that contains the regulatory system capable of interacting with or associating with cells of higher organisms;
c. an effector chemical compound that is a salicylate or salicylate derivative that can be transported through the associated higher organism and activate or repress the expression of the modified host microorganism.

2. The method claimed in claim 1, wherein at least one of the regulatory proteins is controlled by salicylate, anthranylate, 2-acetyl salicylate, 4-chloro-salicylate, 5-chloro-salicylate, 3,5-dichloro-salicylate, 5-methoxy-salicylate, benzoate, 3-methyl-benzoate, 2-methoxy-benzoate, 3-methyl-salicylate, 4-methyl-salicylate, 5-methyl-salicylate, any other salicylate derivative that conserves the carboxylic C-1 group in the aromatic ring, or mixture of the same.

3. The method claimed in claim 2, wherein the regulatory system consists of at least one regulatory protein that belongs to the LysR/NahR family of regulators.

4. The method as claimed in claim 2, wherein the regulatory system consists of at least one derivative of the Xy1S/AraC family of regulators.

5. The method as claimed in claim 2, wherein the regulatory system consists of a regulatory protein belonging to the MarR family of regulators.

6. The method as claimed in claim 2, wherein the regulatory system consists of a system of nahR/Psal expression, or mutants of the same elements.

7. The method as claimed in claim 2, wherein the regulatory system consists of a system of xylS/Pm expression, or mutants of the same elements, capable of responding to any of the chemical compounds cited in claim 2.

8. The method as claimed in claim 2, wherein the regulatory system consists of a genetic cascade circuit that consists of:
a. the transcriptional regulator NahR, or a mutant form of the same, and the transcriptional regulator XylS, or a mutant form of the same, wherein the transcriptional regulators are placed in hierarchical order in such a way that the transcriptional regulator NahR, or a mutant form of the same, stimulates the expression of transcriptional regulator Xy1S, or a mutant form of the same, and wherein the transcriptional regulator NahR, or a mutant form of the same, and the transcriptional regulator Xy1S or a mutant form of the same, respond to the same inducer;
b. a terminal target promoter, wherein said terminal target promoter is **characterized by** its dose-dependent sensitivity to the transcriptional regulator Xy1S, or a mutant form of the same.

9. A method as claimed in claim 1 wherein the cell capable of associating with higher organisms and containing the system regulating heterologous gene expression is a prokaryotic cell.

10. A method as claimed in claim 9 wherein the prokaryotic cell is a gram-negative bacteria.

11. A method as claimed in claim 10 wherein because the gram-negative bacterial cell is a bacteria of the genus Salmonella.

12. A method as claimed in any of the above claims 9 to 11 that has been developed to control the expression of therapeutic proteins or a diagnostic protein in an attenuated bacteria associated with cells of higher organisms.

13. A method as claimed in claim 12 wherein the expression of a heterologous antigen in an attenuated bacteria associated with cells of higher organisms is controlled for use as a recombinant live vaccine.

14. A method as claimed in claim 12 wherein the expression of at least one antitumoral protein that has a certain tropism for tumoral cells in a higher organism is controlled in an attenuated bacteria associated with cells of said higher organism.

15. The method as claimed in claim 14 wherein the antitumoral protein is selected from a group consisting of an interleukin, cytokine, toxin, cytotoxic protein, or antiangiogenic protein.

16. The method as claimed in claim 9 wherein the bacteria also contains a regulatory system sensitive to salicylate or its derivatives and a target promoter, which controls the expression of a suicide gene that encodes a product toxic to the host cell.

17. The method is used as claimed in claim 9 to study the genes implicated in pathogenesis using bacteria with conditional phenotypes.

18. The method is used as claimed in claim 16 for the selective elimination of microorganisms in bioreactors.

19. The method is used as claimed in claim 16 for the selective elimination of cells that can be selectively infected with a bacteria or virus that produces a toxic process or a biomolecule capable of triggering apoptosis or any other mechanism of cell death in malignant cells.

20. The method used to control the expression of heterologous therapeutic proteins or heterologous diagnostic proteins in microorganisms associated with cells of higher organisms consists of:
a. a bacterial cell that contains a regulatory system that can be controlled by salicylate or salicylate derivative as a chemical effector, which is associated specifically to cells of a higher organism;
b. the induction of the expression of said heterologous therapeutic proteins or heterologous diagnostic proteins by administering salicylate or salicylate derivatives to the higher organism hosting the modified cell.

21. The method as claimed in claim 20 wherein the regulatory system is selected from among the group formed by at least one regulatory protein that belongs to the LysR/NahR family of regulators, at least one derivative of the XylS/AraC family of regulators, at least one regulatory protein that belongs to the family of MarR regulators, at least one nahR/Psal regulatory system, or mutant of the same elements, at least one system of xylS/Pm expression, or mutant of the same elements, a genetic cascade circuit that consists of the transcriptional regulator NahR, or a mutant form of the same, and the transcriptional regulator Xy1S, or a mutant form of the same, or a combination of the above.

22. A method as claimed in any of the above claims 20 to 21 wherein said heterologous therapeutic protein is a heterologous antigen, an antitumoral protein, or mixture of the same.

23. The regulatory system of heterologous gene expression in bacteria is associated with tumoral cells of a higher organism and controlled by salicylate or salicylate derivatives to regulate the expression of at least one antitumoral protein.

24. A use as claimed in claim 23 wherein the antitumoral protein expressed is selected from the group consisting of an interleukin, cytokine, toxin, cytotoxic protein, or antiangiogenic protein.

25. The use of a system regulating heterologous gene expression in bacteria associated with cells of a higher organism that is controlled by salicylate or salicylate derivatives to regulate the expression of a heterologous antigen for use as a recombinant live vaccine.

26. The use of a regulatory system of heterologous gene expression in bacteria associated with cells of a higher organism, as claimed in any of claims 23 to 25, wherein said regulatory system is selected from a group consisting of at least one regulatory protein pertaining to the LysR/NahR family of regulators, at least one derivative of the Xy1S/AraC family of regulators, at least one regulatory protein pertaining to the MarR family of regulators, at least one nahR/Psal regulatory system, or mutants of the same elements, at least one xylS/Pm regulatory system, or mutants of the same elements, a genetic cascade circuit that consists of the transcriptional regulator NahR, or a mutant form of the same, and the transcriptional regulator Xy1S, or a mutant form of the same, or a combination of the above.

27. The modified bacteria that contains the regulatory system used is selected from the group consisting of at least one regulatory protein belonging to the LysR/NahR family of regulators, at least one derivative of the XylS7AraC family of regulators, at least one regulatory protein belonging to the MarR family of regulators, at least one nahR/Psal regulatory system, or mutants of the same elements, at least one system of xylS/Pm expression, or mutants of the same elements, a genetic circuit cascade that consists of the transcriptional regulator NahR, or a mutant form of the same, and the transcriptional regulator Xy1S, or a mutant form of the same, or a combination of the above, in the method of control of heterologous gene expression in microorganisms associated to cells of higher organisms. as claimed in claim 1.
